# EUROPEAN PATENT SPECIFICATION

(11) **EP 0 398 583 B1**
(45) Date of publication and mention of the grant of the patent: **06.04.1994**
(21) Application number: 90305029.2
(22) Date of filing: 10.05.1990
(51) Int. Cl.: A61M 5/142, F04B 43/04, F04B 7/00

(54) **Pump apparatus for biomedical use**
Pumpe zur biomedizinischen Verwendung
Pompe pour utilisation biomédicale

(30) Priority: 11.05.1989 GB 8910843; 21.11.1989 GB 8926314
(43) Date of publication of application: 22.11.1990
(73) Proprietor: Bespak plc, King's Lynn Norfolk PE30 2JJ (GB)
(72) Inventor: Ross, Calvin John, Suffolk, IP27 0RB (GB); Humberstone, Victor Carey, Cambridge, CB2 5BT (GB); Warby, Richard John, Wisbech, Cambridgeshire, P13 2HP (GB)
(74) Representative: Alexander, Thomas Bruce

(56) References cited:
- EP-A- 0 110 276
- EP-A- 0 182 502
- EP-A- 0 278 146
- WO-A-82/01997
- WO-A-85/04813
- WO-A-89/07199
- DE-A- 3 515 848
- US-A- 4 255 088
- US-A- 4 596 575

## Description

This invention relates to pump apparatus for biomedical use and in particular but not exclusively for use in intravenous infusion of liquids to the human body or animal body.

Many medical procedures require the controlled introduction of liquids over a prolonged period into the human or animal body for example where a patient is to receive a drug, blood products, saline or nutrients etc at a controlled dose rate. Existing pump apparatus for such applications includes syringe pumps, drip monitors and peristaltic pumps each of which suffer the disadvantages of being expensive, complex to set up and operate and are generally inhibiting of patient movement. It is also known to introduce liquids into plants in a controlled manner in situations where it is inconvenient to have complex pump apparatus relying on external power supplies.

It has also been proposed in DE-2920975 for example to have a peristaltic pump within a housing which also contains means for controlling and actuating the pump so that the apparatus is self-contained and portable and may for example be attached to the body receiving an infusion.

A disadvantage of such peristaltic pumps is that the rate of flow at which liquid is delivered by the pump cannot be accurately controlled. Typically flow rate is measured by counting revolutions of the peristaltic roller mechanism and errors of up to 15% are typical in measured flow rate. A further difficulty is that, in the event of occlusion of the infusion flow path, known pumps and particularly syringe pumps are generally capable of building-up liquid pressure to a dangerous level. It is also difficult to effectively detect the presence of air bubbles within known pumps and typically a bubble detector relies on the use of sensors responsive to the difference in refractive index between air and the liquid being pumped. The presence of air bubbles is extremely dangerous during infusion and a disadvantage of known pumps is that any air bubble will easily pass through the pump.

It is also known from US-A-4596575 to provide an implantable pump apparatus in which a pump chamber receiving an incompressible liquid has a flexible wall movable by actuation of a piezoelectric transducer, the chamber having inlet and outlet valves which are electromagnetically actuated poppet valves. The incompressible liquid is displaced by pump action to dispense an infusion liquid from a collapsible reservoir.

According to the present invention there is disclosed pump apparatus for use in pumping a liquid for infusion into the human or animal body, the apparatus comprising a pump body defining a pump chamber, an inlet duct communicating the pump chamber for connection to a source of an infusion liquid to be pumped whereby in use the liquid fills the pump chamber, an outlet duct communicating with the chamber for the delivery of the liquid when pumped, actuating means operable to vary the colume of the pump chamber by movement of a flexible wall of the pump chamber to pump the liquid therefrom, first and second valves located in the inlet and outlet ducts respectively, the actuating means comprising a piezoelectric element having at least one sensor electrode and electronic circuit means operable to energise the piezoelectric element in pulsed manner, characterized by each valve comprising a non-return valve operable in response to pressure or suction generated in the pump chamber and the at least one sensor electrode sensing the amplitude of deflection of the flexible wall wherein the electronic means includes detection means comparing the sensed amplitude of deflection with at least one threshold level to provide an indication of abnormal operation of the pump apparatus consistent with occlusion or bubble formation in a volume of the liquid defined by the pump chamber and the inlet and outlet ducts.

An advantage of such pump apparatus is that during normal pumping operation the valves do not require actuation from an external source since they respond to pressure or suction in the pump chamber.

Preferably the pump apparatus further comprises priming means selectively operable between a normal operating condition in which the valve members are biassed into a seated position and a priming condition in which the valve members are movable into an unseated position to allow priming of the pump chamber with fluid.

An advantage of such priming means is that prior to normal pumping operation the valve members can be unseated to allow a free flow of fluid through the pump chamber to fully flush and prime the pump apparatus.

The pump apparatus may comprise electrical circuit means operable to control and energise the actuating means whereby the pump apparatus is self-contained, the actuating means comprising a piezoelectric transducer operable to deform the flexible wall of the pump body and wherein the electrical circuit means is located in a housing which is detachable from the pump body and the housing is connected in use to the pump body by releasable connecting means.

An advantage of such an arrangement is that the pump body can be made a disposable item whilst the housing can be reusable. Electrical circuitry can therefore be retained within the housing and reused whereas components of the apparatus such as the chamber and valves which make contact with the liquid to be pumped are made disposable. A further adavantage is that the pump body can be sterilised prior to use by conventional sterilisation techniques which might not be tolerated by the electrical components within the housing.

Preferably the transducer is located in the housing at a location which overlays the flexible wall of the pump body in use when the housing and pump body are connected in operable relationship and wherein the apparatus includes biassing means biassing the wall into operative engagement with the transducer.

The flexible wall of the pump body then flexes in unison with the transducer so that transducer movement can be sensed and calibrated to provide an accurate measure of the amplitude of each pump actuation.

The biassing means may comprise an annular membrane portion of the flexible wall connected peripherally to a rigid portion of the flexible wall and having a shape memory such that when the pump body is operatively connected to the housing the rigid portion is biassed into engagement with the transducer.

The body may be formed integrally with a reservoir receiving in use a supply of fluid, the reservoir being connected in communication with the inlet duct.

An advantage of such an arrangement is that the pump body and the reservoir containing the supply of fluid can together be supplied as a replaceable item.

The housing preferably includes a reservoir compartment receiving the reservoir when the housing and the body are operatively connected and closure means for the reservoir compartment, the priming means being actuated by a manually operated handle mounted on the housing and wherein the handle and the closure means interlock to prevent access to the reservoir compartment when the handle is in a position corresponding to actuation of the priming means to give normal operating condition of the magnetic means.

Preferably the detection means includes occlusion detection means operable to compare the sensed amplitude of deflection against a lower threshold level and indicating the presence of an occlusion when the sensed amplitude is less than the lower threshold level.

An advantage of such an arrangement is that occlusion can be instantly detected and an alarm signal used to generate an audible or visible warning or to turn off the pump.

Preferably the electronic circuit means includes bubble detection means operable to compare the sensed amplitude of the transducer actuation against an upper threshold level and indicating the presence of a bubble when the sensed amplitude is more than the upper threshold level.

The presence of a bubble in the pump chamber can be instantly detected in this way since collapse of an air bubble under pressure will result in over travel of the flexible wall of the pump chamber and hence over travel of the piezoelectric transducer beyond its normal operating range.

Preferably the pump apparatus includes flow rate sensing means connected to the electronic circuit means and the circuit means energises the piezoelectric transducer at a frequency which is variable to maintain the sensed flow rate at a predetermined level.

Preferred embodiments of the present invention will now be described by way of example only and with reference to the accompanying drawings of which:-
Figure 1 is a side elevation of pump apparatus in accordance with the present invention;
Figure 2 is a sectioned plan view of the apparatus of Figure 1;
Figure 3 is a sectioned end elevation of the apparatus of Figures 1;
Figure 4 is a sectional elevation of part of an alternative apparatus showing detail of the piezoelectric transducer;
Figure 5 is an enlarged view of the tranducer of Figure 4;
Figure 6 is a plan view of the transducer of Figures 4 and 5;
Figure 7 is an underneath view of the transducer of Figures 4 to 6;
Figure 8 is a plan view of a further alternative pump apparatus having a key pad and display;
Figure 9 is an elevation of a further alternative pump apparatus connected to a supply pack and flexible tube;
Figure 10 is an elevation of the apparatus of Figure 9 showing a housing of the apparatus being presented to a disposable body of the apparatus prior to an infusion;
Figure 11 is a sectional elevation of a further alternative pump apparatus;
Figure 12 is a plan view of the pump apparatus of Figure 11;
Figure 13 is an end view partially in section of the apparatus of Figures 11 and 12;
Figure 14 is a perspective view of an alternative pump apparatus having an integral reservoir; and
Figure 15 is a perspective exploded view of the apparatus of Figure 14.

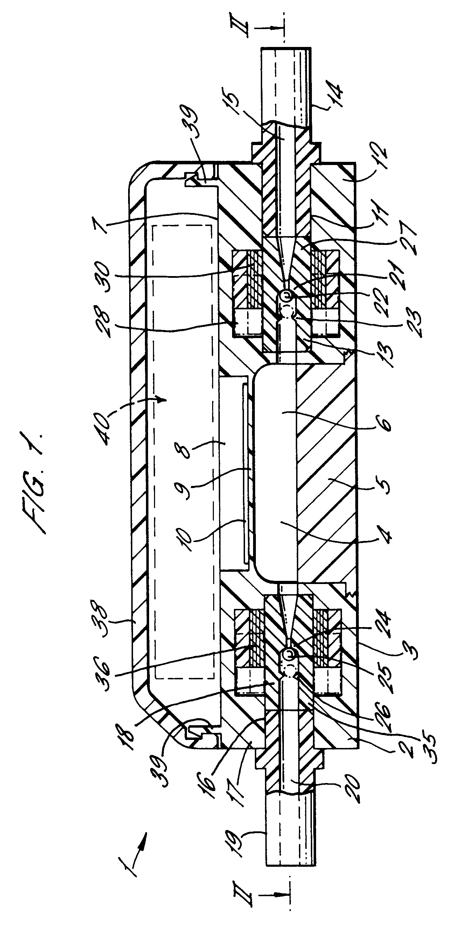

Figure 1 shows apparatus 1 for use in the delivery of a drug intravenously to a patient. Apparatus 1 comprises an elongate body 2 moulded from a plastics material. The body 2 is of rectangular cross-section and has a bottom surface 3 in which is formed a cylindrical recess 4. A screw-threaded plug 5 fits sealingly into the recess 4 to define a chamber 6 within the recess. The plug 5 is moulded from polystyrene containing 40% glass fibres.

The body 2 has a top surface 7 in which is formed a second cylindrical recess 8 such that a thin membrane 9 is defined between the first and second recesses 4 and 8 and forms a flexible wall to the chamber 6.

A piezoelectric membrane 10 overlays the flexible membrane 9, the respective membranes 9 and 10 being in intimate contact such that any flexure of the piezoelectric membrane is accompanied by conformal flexure of the flexible membrane.

A first longitudinal bore 11 is formed in a first end 12 of the body 2 and receives a first non-return valve 13 and a first cylindrical connector 14 defining an inlet duct 15 communicating with the chamber 6.

A second bore 16 is formed longitudinally in the second end 17 of the body 2 and receives a second non-return valve 18 and a second tubular connector 19 defining an outlet duct 20 communicating with the chamber 6.

The first non-return valve 13 comprises an annular valve seat 21 through which the inlet duct 15 extends and includes a spherical steel valve member 22 coated in polytetrafluorethylene (PTFE). The valve member 22 is movable longitudinally within the inlet duct to an extent limited in a direction away from the seat 21 by projections 23 which project radially inwardly of the duct.

The second non-return valve 18 is similarly provided with a valve seat 24, a spherical valve member 25 and projections 26. The first and second non-return valves 13 and 18 are arranged such that in each case flow of fluid in a direction from the outlet duct to the inlet duct is prevented by sealing engagement of the respective valve member 21, 25 with the respective seat 21, 24.

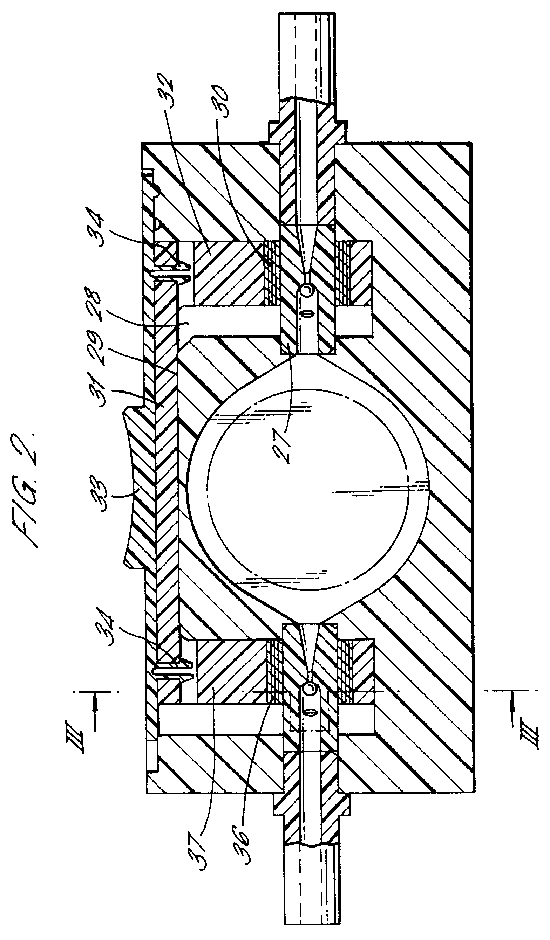

The first non-return valve 13 has a cylindrical valve body 27 which intersects a first aperture 28 which extends transversely into the body 2 from a right-hand side surface 29 of the body 2 as seen in Figure 2. A first annular magnet 30 is coaxially mounted on the first valve body 27 and is axially slidable relative to the valve body between the normal position as shown in Figure 1 and a pump priming position shown in broken lines. In its normal position the centre of magnet 30 lies between the seat 21 and the first end 12 of the body 2. In its priming position the centre of magnet 30 lies on the other side of the seat 21 adjacent the projections 23. A slider plate 31 is mounted in contact with the right-hand side surface 29 so as to be longitudinally slidable relative to the body 2 and is connected to the first annular magnet 30 by a first arm 32. A handle 33 is connected to the slider plate 31 by means of snap fit connectors 34.

The second non-return valve 18 similarly has a cylindrical valve body 35 on which a second annular magnet 36 is axially slidable, the second magnet being connected to the slider plate 31 by a second arm 37. The first and second magnets 30 and 36 are thereby movable between respective normal and priming positions by actuation of handle 33.

A housing 38 is detachably connected to the body 2 by means of snap fit connectors 39 so as to overlay the top surface 7. The housing 38 contains a control unit 40 represented schematically by broken lines as occupying the housing.

The control unit 40 includes releasable connecting means (not shown) operable to make electrical connection with the piezoelectric membrane 10. The control unit 40 contains a battery (not shown) and programmable circuitry for energising the piezoelectric membrane 10 so that the apparatus 1 is self-contained.

In use the first connector 14 is connected to a supply of fluid and the slider plate 31 is manually moved into a pump priming position (to the left in Figure 1). In this position the valve members 22 and 25 are unseated from the respective valve seats 21 and 24 by magnets 30 and 36 respectively. Fluid is then able to flow through the inlet duct 15, the chamber 6 and the outlet duct 20 so as to prime the apparatus 1.

The slider plate 31 is returned to its normal position (to the right in Figure 1) in which the valve members 22 and 25 are held in contact with respective valve seats 21 and 24 by magnets 30 and 36 respectively thereby preventing flow of fluid through the apparatus.

To commence an infusion the second connector 19 is connected to a suitable fluid filled cannula in a manner which avoids introduction of air into the cannula or the apparatus 1.

Infusion is commenced by turning on the control unit 40 which is programmed to deliver actuating electrical pulse sequences to the piezoelectric membrane 10. Each actuating pulse sequence consists of a positive pulse followed by a negative pulse of the same amplitude and duration and the frequency with which pulse sequences are generated is varied by the control unit 40 in accordance with the required flow rate.

At each positive actuating pulse the piezoelectric membrane 10 flexes in a direction towards the flexible membrane 9 thereby causing corresponding flexure of the membrane 9 which results in a decrease of the volume of chamber 6. Fluid in chamber 6 is thereby pressurised and the second non-return valve 18 is opened under fluid pressure, the valve member 25 being unseated from its valve seat 24 and held against projection 26 in a position which allows fluid flow from the chamber 6 through the outlet duct 20.

At the end of the positive actuating pulse the membrane 10 relaxes to its initial position as shown in Figure 1 as does the flexible membrane 9 thereby restoring the chamber 6 to its original volume and creating suction within the chamber 6. The second non-return valve 18 is closed by this suction as the second valve member 25 seats against the valve seat 24 and simultaneously the first non-return valve 13 is opened as the valve member 22 is unseated from its valve seat 21 and is held against projections 23 in a position which allows flow of fluid to pass from the inlet duct into the chamber 6.

A negative actuating pulse is then received by the piezoelectric membrane 10 which flexes in a direction away from the flexible membrane 9 thereby causing corresponding flexure of the membrane 9 which results in an increase in the volume of chamber 6. Suction is then created within chamber 6 which draws fluid into the chamber through the first non-return valve 13. At the end of the negative actuating pulse the membrane 10 relaxes to its initial position as shown in Figure 1 as does the flexible membrane 9 thereby restoring the chamber 6 to its original volume and creating pressure within the chamber. This pressure results in a first non-return valve 13 being closed and the second non-return valve 18 being opened accompanied by fluid being pumped under pressure from the chamber to the second non-return valve and out through the outlet duct 20. During the interval between successive actuating pulse sequences the first and second non-return valves 13 and 18 are closed by action of the first and second magnets 30 and 36 which maintain a constant bias of the valve members 21 and 25 towards their respective valve seats 21 and 24.

At each cycle of operation of the apparatus 1 as described above a substantially constant volume of liquid is pumped so that the flow rate at which an infusion proceeds is determined by the frequency of actuating pulses controlled by the control unit 40.

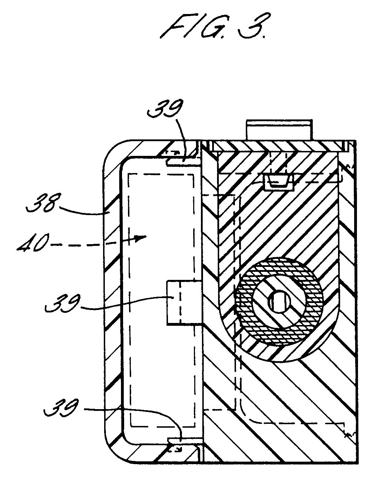

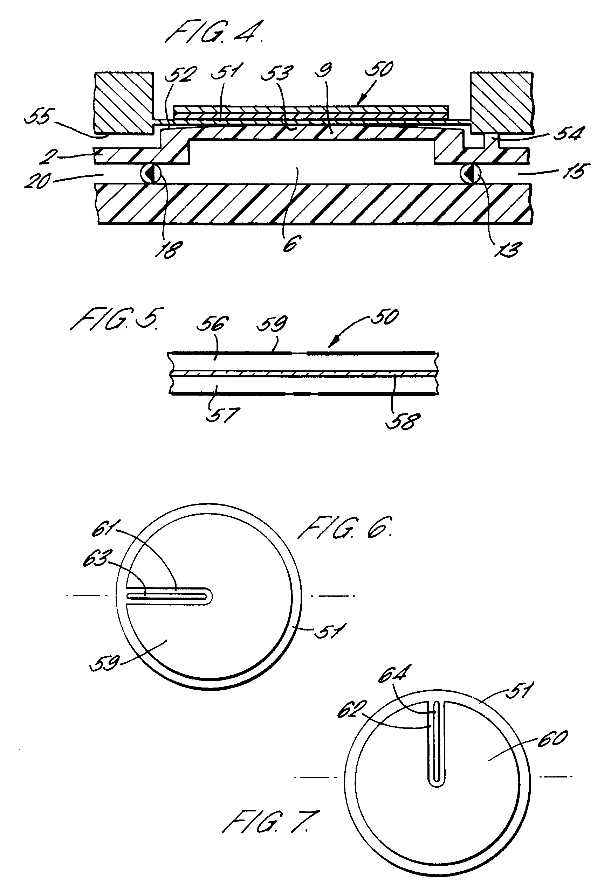

A modification to the apparatus 1 will now be described with reference to Figure 4 and using reference numerals corresponding to those of Figures 1 to 3 for corresponding elements where appropriate.

In Figure 4 a composite piezoelectric membrane 50 is located in the housing 38 and is attached to a thin flexible membrane 51 forming an external surface of the housing.

The housing 38 is detachably connected to a body 2 connected to an inlet duct 15 and an outlet duct 20 via first and second non-return valves 13 and 18 respectively (represented schematically in Figure 4).

The body 2 includes a flexible membrane 9 forming a flexible wall to the chamber 6 and the body membrane 9 has a domed external surface 52 having an apex 53 which maintains contact with the planar housing membrane 51.

Three locating lugs 54 project from the body 2 in circumferentially spaced positions relative to the flexible body membrane 9 and abut with an annular surface 55 of the housing 38 which is connected to the housing membrane 51 such that the housing membrane 51 is peripherally supported at a fixed distance relative to the body 2.

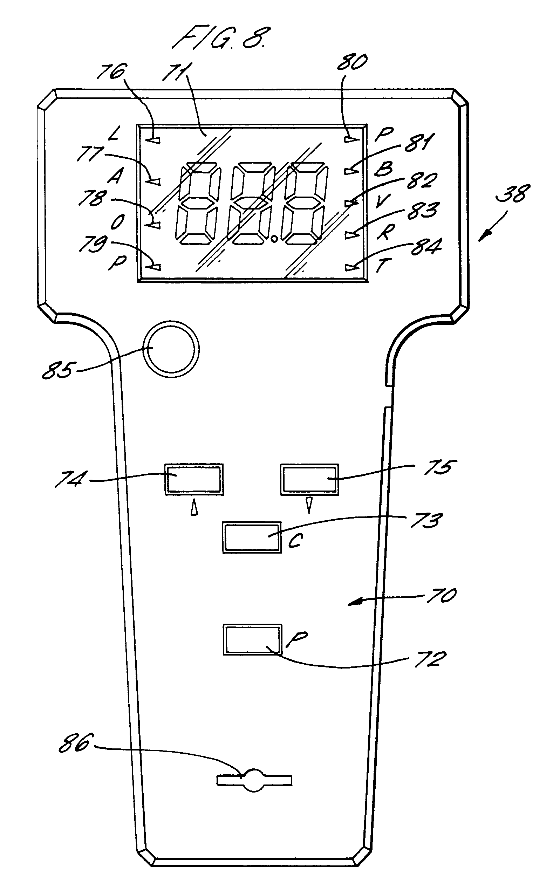

The structure of the composite piezoelectric membrane 50 is shown in Figure 5 in enlarged scale. The composite piezoelectric membrane 50 comprises upper and lower ceramic discs 56 and 57 respectively separated by a brass disc 58. Upper and lower metallic drive electrodes 59 and 60 respectively are formed on the upper and lower surfaces of the upper and lower discs 56 and 57 respectively and are electrically connected to the control unit 40 so as to receive actuating pulses resulting in flexure of the membrane 50 by electrical polarisation of the ceramic discs 56 and 57.

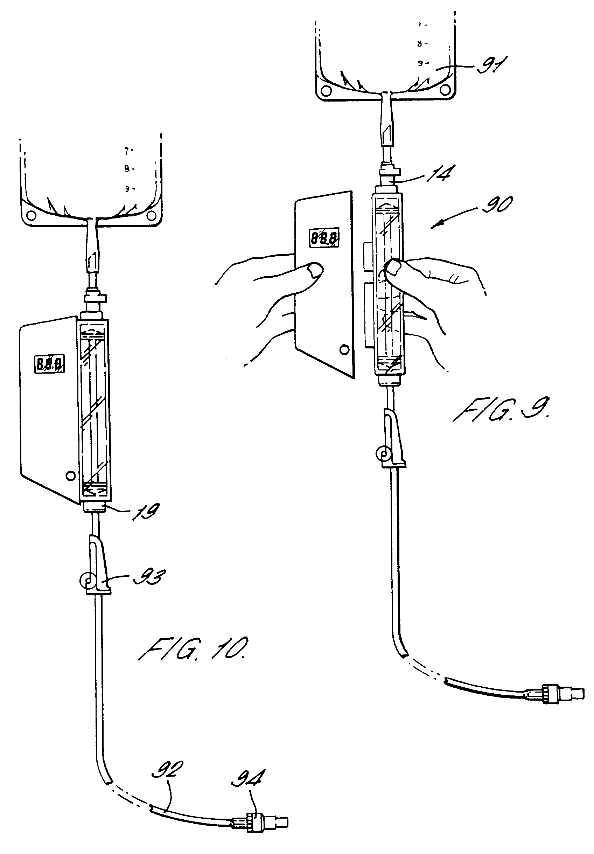

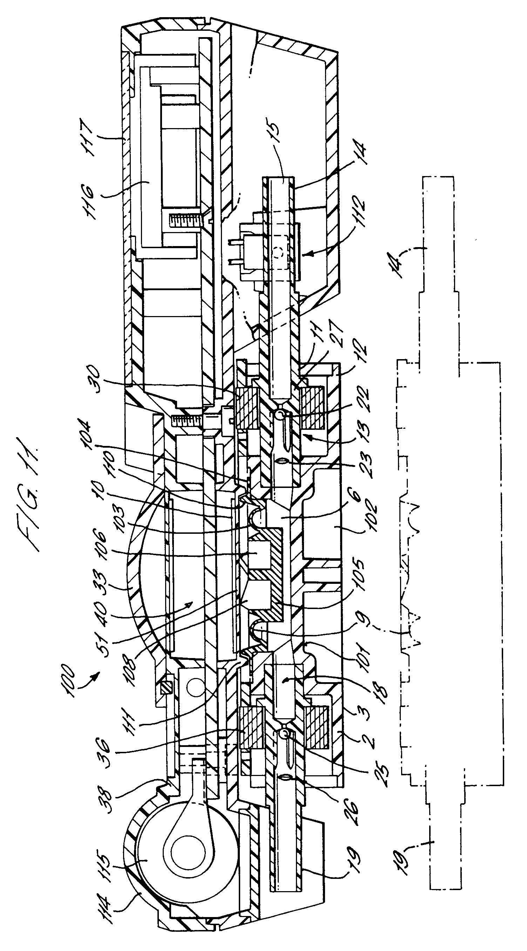

The upper and lower drive electrodes 59 and 60 each include radially extending cutouts 61 and 62 respectively which are angularly spaced by 90° as seen from Figures 6 and 7 and upper and lower sense electrodes 63 and 64 respectively extend radially within the respective cutouts 61 and 62 so as to be electrically isolated from the drive electrodes.

The upper and lower sense electrodes 63 and 64 are connected to the control unit 40 to provide feedback as to the extent of flexure of the piezoelectric membrane 50 and hence feedback as to the corresponding flexure of the body membrane 9. The control unit 40 is provided with circuitry (not shown) deriving a signal representing the amplitude of flexure of the membranes 50 and 9 and comparing the signal with maximum and minimum threshold levels to generate alarm signals if the signal is not within these upper and lower limits. In this way the apparatus 1 is able to indicate fault conditions arising either from the presence of air bubbles in the pump chamber 6 or occlusion of the downstream infusion path with respect to the pump chamber.

The effect of an air bubble being formed within the pump chamber 6 is that flexure of the membrane 9 so as to reduce the volume of the pump chamber would result in reduction in the volume of the air bubble since air is readily compressible whereas the surrounding liquid is highly incompressible. Consequently the extent of travel through which the membrane 9 flexes will be greater than during a normal pump actuation in the absence of any air bubble and consequently the signal amplitude derived from the sense electrodes 63 and 64 will be greater than usual and can be detected as such by comparison with a predetermined upper threshold level.

Similarly the effect of an occlusion downstream of the pump chamber 6 for example in the outlet duct 20 would be that liquid would be prevented from leaving the chamber 6 on being compressed by flexure of the membrane 9 and the incompressibility of the liquid would limit flexure of membrane 9 to less than during a normal pump actuation. The amplitude of the signal derived from the sense electrodes 63 and 64 would therefore be less than usual and can be detected as such by comparison with a predetermined lower threshold level.

During normal operation the output signal derived from the sense electrodes 63 and 64 provides a measure of flow the volume of liquid metered at each actuation of the apparatus 1. The control unit 40 is programmed to deliver a predetermined flow rate and adjusts the actuating pulse frequency to achieve the required flow rate according to the signal amplitude. In the above examples the range of actuating pulse frequency during normal operation is between 25 and 50 Hz and the volume of the pump chamber is one microlitre.

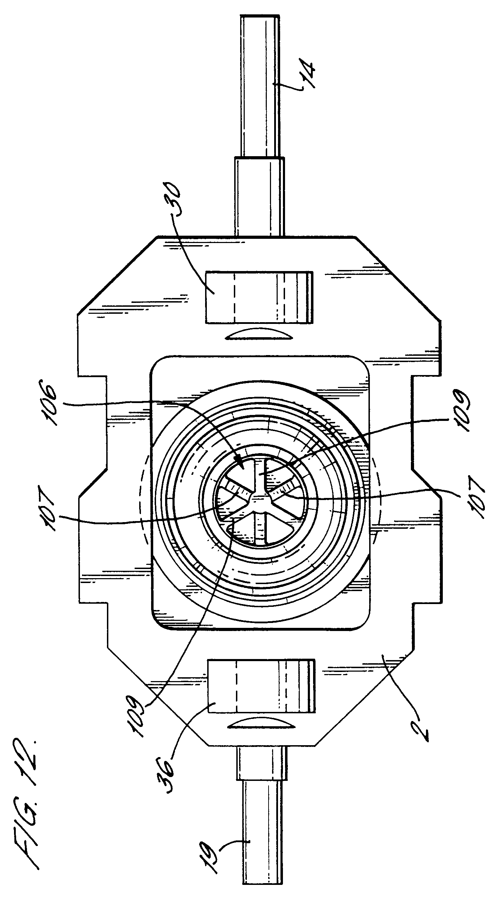

The housing 38 may be of any convenient external shape providing that it is adapted to mate with the body 2 and may for example include a keypad 70 and display 71 as shown in Figure 8. The keypad 70 has a PUMP button 72, a CONFIRM button 73, a STEP UP button 74 and STEP DOWN button 75 for the input of data to the control unit 40.

The display 71 is of a liquid crystal type and further includes light emitting diode indicators 76 to 84. An electro acoustic transducer 85 capable of generating an audible warning is also mounted on the housing 38.

A keyhole 86 for receiving an actuating key is also provided in the housing 38.

In use a user primes the apparatus 1 before connection of the body 2 to the housing 38 as described above. The housing 38 is then clipped to the body 2 after which the control unit 40 automatically carries out some self-checking steps to establish whether good contact has been made with the body 2, whether the battery voltage is adequate, that the display and audible warning 85 are in order, that there is no air in the chamber 6 and that there is no occlusion in the apparatus 1 (these latter checks requiring some initial actuations of the piezoelectric membrane 10).

The self-check as to the correct operation of the display 71 and audible transducer 85 is carried out by the control unit illuminating all of the indicators 76 to 84 and sounding the transucer 85 at which the user should respond by pressing the CONFIRM button 73. On receiving this confirmation the control unit 40 ceases to illuminate the indicators 76 to 84 and turns off the transducer 85. Indicator 76 which is designated as PROGRAMME indicator is then made to flash on and off to indicate to the user that the control unit 40 is ready to receive programming instructions. The user then inserts a key into the keyhole 86 to actuate a key switch (not shown) permitting entry of programme data. At this point the indicator 83 which is designated as VOLUME IN MILLILITRES is illuminated and the user enters the required data for the volume of infusion by scrolling the numerical display 71 using the STEP UP or STEP DOWN key 74, 75 as required. When the required number is indicated by the display 71 the data is entered by pressing the CONFIRM button 73.

A similar procedure is then followed to enter further data prompted by illumination of indicator 83 designated as RATE in millilitres per hour, indicator 82 designated as BOLUS and indicator 84 which is designated as TIME INTERVAL.

The user can therefore programme the duration and rate of an infusion together with the maximum volume required. The bolus setting indicates a preset bolus volume which can be delivered if the need should arise during the infusion i.e. if a higher flow rate is required for a short duration as in the case of the need to administer a pain killer at an increased dosage.

On completion of programming the key is removed and the PUMP button 72 pressed to commence the infusion. Removal of the key prevents a patient from changing any of the programmed settings. An audible warning is generated by the transducer 85 five minutes before completion of the infusion in order to alert an operator that completion is imminent. Completion of the delivery of the required volume is displayed by the control unit 40 turning off the indicator 79 denoted as a PUMPING indicator and an audible warning is sounded by the transducer 85.

Indicator 77 is designated AIR BUBBLES and will be lit if an air bubble is detected. An audible warning is also produced using transducer 85. Similarly illumination indicator 78 corresponds to detection of occlusion. The alarm signal generated by the transducer 85 can be silenced by an operator pressing the CONFIRM button 73.

Indicator 76 is designated as a LOW BATTERY indicator.

On completion of the infusion the housing 38 is unclipped from the body 2 and the body 2 is disposed of as waste. An important advantage of the pump apparatus of the present invention when used in medical applications is that whereas the electronic control apparatus within the housing 38 is reusable the body 2 is disposable and of relatively low cost so that the body can be repeatedly and frequently renewed. This is important because of the micro biological hazards of infusion therapy and the need to comply with administration procedures requiring infusion apparatus to be changed at regular intervals.

The control unit 40 is also provided with a separate rechargeable battery which is connected so as to maintain programme memory during periods in which the control unit is not connected to its normal power supply battery. The control unit may then be programmed to maintain a log of each use of the apparatus over an extended period of time. The control unit may also be programmed to display on demand an indication of the total time for which a particular disposable pump body 2 has been in use.

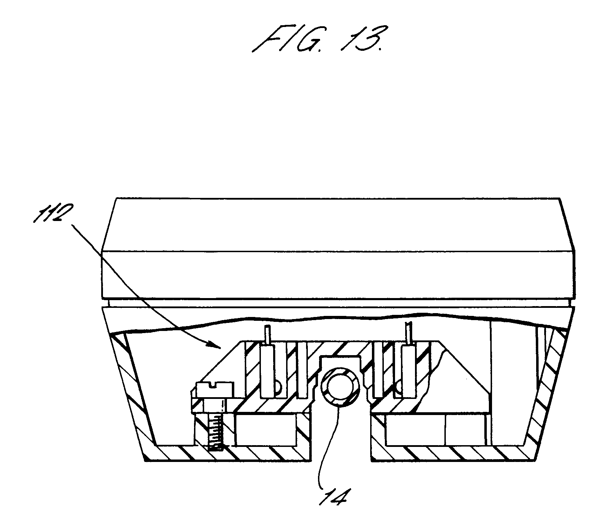

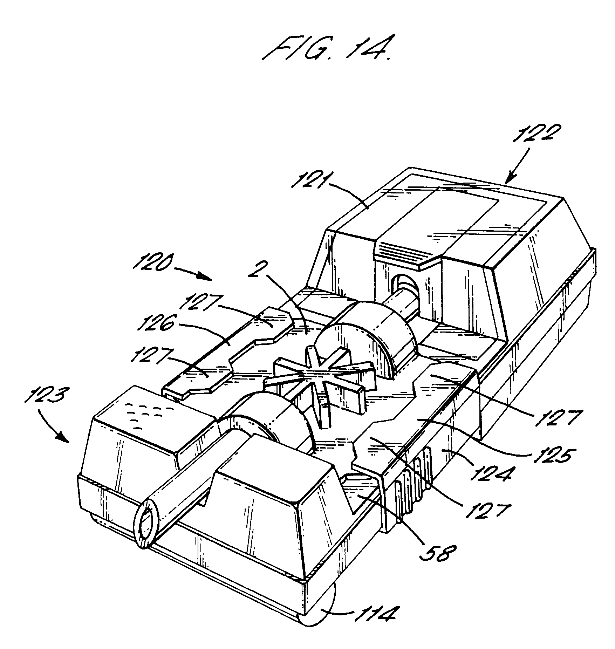

In Figures 9 and 10 apparatus 90 of alternative external shape is illustrated, corresponding reference numerals to those of apparatus 1 being used where appropriate.

In Figure 9 a housing 38 is being presented to a disposable body 2 prior to an infusion.

In Figure 10 the housing 38 is shown connected to the body 2 during an infusion in which a supply pack 91 is connected to the first connector 14. The second connector 19 is connected to a cannula (not shown) via a flexible tube 92 to which is fitted a shut off valve 93 and a luer connector 94.

The apparatus is powered by a battery which may be located either within the housing or within the pump body. If it is located within the pump body then this has the advantage that if the pump body is a disposable item then a new battery is provided with each successive use with a given housing.

The materials used in the pump body must be selected to be compatible with the liquid to be pumped. This is particularly true of medical applications where medically approved plastics materials are essential.

The apparatus may additionally be provided with means which allow operative connection of the housing only to approved pump bodies so as to exclude for example pump bodies copied by competitors and possibly of an inferior quality. These means may for example include a surface relief holographic element on the pump body which is scanned by a light detector in the housing. Simple mechanical interlocks may also be provided.

The apparatus may be modified to include a further bubble detector of known type such as a sensor responsive to optical refraction of the contents of the inlet duct. This provides an additional safeguard against bubbles entering the apparatus and can be used to generate warnings in the same manner as the bubble detection circuit incorporated in the control unit 40 and responsive to flexure of the piezoelectric membrane.

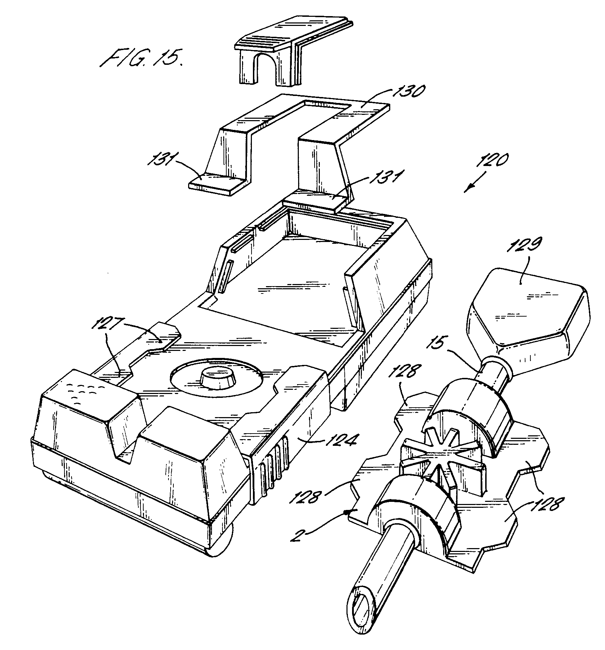

Figures 11, 12 and 13 show a further alternative apparatus 100 which includes many features in common with the apparatus described with reference to the previous Figures and therefore corresponding reference numerals for corresponding elements are used where appropriate. Apparatus 100 has a body 2 formed of a transparent medically approved plastics material. The body 2 is intended to be a disposable item and is shown in the upper portion of Figure 11 connected to a non-disposable housing 38 and is also shown in outline in the lower portion of Figure 11 in which it is separated from the housing.

The body 2 has a bottom surface 3 having a circular central portion 101 which is strengthened by radially extending stiffening ribs 102. Above the central portion 101 a chamber 6 is defined within the body 2 and the volume of the chamber is variable by flexure of a flexible membrane 9 forming a flexible wall to the chamber 6. The flexible membrane 9 comprises an annular flexible portion 103 of semi-circular cross-section connected between an outer support ring 104 and a central rigid disc 105. A spider member 106 is formed integrally with the disc 105 such that the spider member and disc together form a rigid assembly which is movable vertically relative to the support ring 104 by flexure of the flexible annular portion 103.

As seen more clearly in Figure 12 the spider member 106 includes three radially extending arms 107 each including a vertically extending projection 108 of triangular elevation. Between the arms 107 three further arms 109 are also provided, these further arms being similar to arms 107 but not being provided with features corresponding to the projections 108.

The support ring 104 is bonded to the body 2 to thereby seal the chamber 6 and the support ring also includes an externally projecting annular raised lip 110.

The housing 38 is connectable to the body 2 such that an annular flange 111 locates around the lip 110. The housing includes a membrane 51 within the annular flange 111 which in the connected configuration as shown in Figure 11 is positively held in contact with the projections 108 of the spider member 106. A piezoelectric membrane 10 overlays the housing membrane 51 such that the membrane 51 is flexible in unison with the piezoelectric membrane.

The annular flexible portion 103 has a shape memory such that whenever body 2 is operatively connected to the housing 38 the spider member 106 is biassed into contact with the housing membrane 51 with a force equivalent to a weight of 500 grammes. The disc 105 therefore moves in unison with the piezoelectric membrane 10.

The body 2 is provided with first and second tubular connectors 14 and 19 respectively which communicate with the chamber 6 via first and second valves 13 and 18 respectively of similar type to those of the apparatus 1. The apparatus 100 further includes an air bubble detector 112 which is connected to the housing 38 such that when the body 2 is connected to the housing the first tubular connector extends through the bubble detector. The bubble detector 112 is of known type comprising a light source on one side of the tubular connector 14 and a light sensor on the opposite side of the connector and sensitive to change in the refractive index of the contents of the duct 15 defined by the tubular connector. The detector 112 supplements the means of bubble detection referred to above with reference to apparatus 1.

Housing 38 includes a control unit 40 mounted on a printed circuit board 113 and further includes a battery compartment 114 containing a replaceable battery 115. The control unit 40 is also connected to an auxiliary battery (not shown) which maintains the memory functions of the control unit during replacement of the replaceable battery 115.

The housing 38 further includes a liquid crystal display 116 which is viewable through a transparent window 117.

The housing 38 includes a handle 33 which is connected to first and second annular magnets 30 and 36 respectively and arranged so as to be slidable longitudinally of the housing between a normal position as shown in Figure 11 and a priming position (not shown) in which the magnets are moved to the left of Figure 11 so as to unseat valve members 22 and 25 during a priming operation.

A further alternative apparatus is shown in Figures 14 and 15 where corresponding reference numerals to those of previous Figures are used where appropriate for corresponding elements.

The apparatus 120 has a housing 58 with a reservoir compartment 121 at a first end 122 of the housing. A battery compartment 114 is located at a second end 123 of the housing.

A sliding handle 124 is longitudinally slidably mounted on the housing 58, the handle being generally of U-shaped cross-section with inturned edge portions 125 and 126 each having projecting tabs 127 which overlay and thereby hold in place the disposable body 2. The apparatus 120 of Figures 14 and 15 is shown in a different orientation to the apparatus of previous Figures i.e. such that the body 2 is uppermost.

As seen in Figure 15 the body 2 includes laterally projecting lugs 128, the respective lugs 127 and 128 being longitudinally spaced so as to be inter-digitated during connection of the body 2 and the housing 58. A reservoir 129 comprising a collapsible plastic sac is connected to the inlet duct 15 . When the disposable body 2 is connected to the housing 58 the reservoir 129 is concealed within the reservoir compartment 121 which is accessible via a laterally slidable reservoir cover 130. The reservoir cover 130 includes laterally projecting lugs 131 which in the normal operating position of the handle 124 are overlaid by lugs 127 such that the reservoir cover cannot be removed.

In order to load a fresh disposable body 2 into the housing 58 the handle 124 must first be moved fully towards the second end 123 of the housing and the reservoir cover 130 removed. The disposable body and reservoir 129 can then be loaded on to the housing 58 and the reservoir cover 120 replaced. The handle 124 is then moved fully towards the first end 120 of the housing 58 such that the lugs 127 overlay the lugs 128 of the disposable body 2 and also overlay the lugs 131 of the reservoir cover. Both the body 2 and the reservoir cover 131 are then held in position.

During this procedure the initial position of the handle 124 is such that the magnets 30 and 36 are in their priming position (as described with reference to Figure 1). After movement of the handle towards the first end 121 the magnets 30 and 36 are moved to their normal operating position in which the first and second non-return valves 13 and 18 open and close in response to fluid pressure.

In an alternative embodiment the spherical valve members may be replaced by a bullet-shaped valve member.

The non-return valves may alternatively be moved to their priming positions by an electromagnet.

The reservoir may carry an identifying coded element which can be read by a suitable detector of the housing such that the control unit 40 is automatically provided with data when a fresh body 2 complete with drug reservoir 129 is loaded to the housing. The control unit 40 may be programmed on receipt of this data to deliver the drug in accordance with predetermined parameters without the need for operator input. The control unit 40 may also record and display the total dose of drug delivered from a given reservoir and generate an alarm when the supply is nearly exhausted.

## Claims

1. Pump apparatus (1,100,120) for use in pumping a liquid for infusion into the human or animal body, the apparatus comprising a pump body (2) defining a pump chamber (6), an inlet duct (15) communicating with the pump chamber for connection to a source (129) of an infusion liquid to be pumped whereby in use the liquid fills the pump chamber, an outlet duct (20) communicating with the chamber for the delivery of the liquid when pumped, actuating means (10,50) operable to vary the volume of the pump chamber by movement of a flexible wall (9) of the pump chamber to pump the liquid therefrom, first and second valves (13,18) located in the inlet and outlet ducts respectively, the actuating means comprising a piezoelectric element (10,50) having at least one sensor electrode (63,64) and electronic circuit means (40) operable to energise the piezoelectric element in pulsed manner, characterized by each valve comprising a non-return valve operable in response to pressure or suction generated in the pump chamber and the at least one sensor electrode (63,64) sensing the amplitude of deflection of the flexible wall (9), wherein the electronic circuit means includes detection means comparing the sensed amplitude of deflection with at least one threshold level to provide an indication of abnormal operation of the pump apparatus consistent with occlusion or bubble formation in a volume of the liquid defined by the pump chamber and the inlet and outlet ducts.

2. Pump apparatus as claimed in claim 1 wherein the detection means includes occlusion detection means operable to compare the sensed amplitude of deflection against a lower threshold level and indicating the presence of an occlusion when the sensed amplitude is less than the lower threshold level.

3. Pump apparatus as claimed in claim 1 or 2 wherein the detection means includes bubble detection means operable to compare the sensed amplitude of the deflection against an upper threshold level and indicating the presence of a bubble when the sensed amplitude is more than the upper threshold level.

4. Pump apparatus as claimed in any of claims 1 to 3 including flow rate sensing means connected to the electronic circuit means and wherein the circuit means energises the piezoelectric element at a frequency which is variable to maintain the sensed flow rate at a predetermined level.

5. Pump apparatus as claimed in any preceding claim wherein the electrical circuit means is located in a housing (38) which is detachable from the pump body and the housing is connected in use to the pump body by releasable connecting means (39).

6. Pump apparatus as claimed in any preceding claim wherein the piezoelectric element is located in the housing at a location which overlays the flexible wall of the pump body in use when the housing and pump body are connected in operable relationship and wherein the apparatus includes biassing means (103) biassing the wall into operative engagement with the transducer.

7. Pump apparatus as claimed in claim 6 wherein the biassing means comprises an annular membrane portion (103) of the flexible wall connected peripherally to a rigid portion (105) of the flexible wall and having a shape memory such that when the pump body is operatively connected to the housing the rigid portion is biassed into engagement with the transducer.

8. Pumping apparatus as claimed in any of claims 5 to 7 wherein the body is formed integrally with a reservoir (129) receiving in use a supply of fluid, the reservoir being connected in communication with the inlet duct.

9. Pumping apparatus as claimed in claim 8 wherein the housing includes a reservoir compartment (121) receiving the reservoir when the housing and the body are operatively connected and closure means (130) for the reservoir compartment, the apparatus further comprising priming means selectively operable to bias the first and second valves into the open condition to allow priming of the chamber, the priming means being actuated by a manually operated handle (33) mounted on the housing and wherein the handle and the closure means interlock to prevent access to the reservoir compartment when the handle is in a position corresponding to de-actuation of the priming means to give normal operation of the valves.

## Patentansprüche

1. Pumpvorrichtung (1, 100, 120) zur Verwendung beim Pumpen einer Flüssigkeit zur Infusion in den menschlichen oder tierischen Körper, wobei die Vorrichtung umfaßt einen eine Pumpkammer (6) festlegenden Pumpkörper (2), eine mit der Pumpkammer in Verbindung stehende Einlaßleitung (15) zum Anschluß an eine Quelle (129) einer zu pumpenden Infusionsflüssigkeit, wodurch die Flüssigkeit in Betrieb die Pumpkammer füllt, eine mit der Kammer in Verbindung stehende Auslaßleitung (20) zur Abgabe der Flüssigkeit beim Pumpen, Betätigungsmittel (10, 50), welche betreibbar sind, um das Volumen der Pumpkammer durch Bewegung einer flexiblen Wandung (9) der Pumpkammer zum Pumpen der Flüssigkeit aus dieser zu verändern, ein erstes und ein zweites in der Einlaßleitung bzw. der Auslaßleitung angeordnetes Ventil (13, 18), wobei die Betätigungsmittel ein wenigstens eine Sensorelektrode (63, 64) aufweisendes piezoelektrisches Element (10, 50) und ein zum Erregen des piezoelektrischen Elements in pulsierender Weise betreibbares elektronisches Schaltungsmittel (40) umfassen,
dadurch gekennzeichnet, daß
jedes Ventil ein auf in der Pumpkammer erzeugten Druck oder Sog ansprechend betreibbares Rückschlagventil umfaßt und daß die wenigstens eine Sensorelektrode (63, 64) die Amplitude der Auslenkung der flexiblen Wandung (9) erfaßt, wobei das elektronische Schaltungsmittel ein Feststellungsmittel aufweist, welches die erfaßte Amplitude der Auslenkung mit wenigstens einem Schwellenniveau vergleicht, um eine Anzeige eines abnormalen Betriebs der Pumpvorrichtung vorzusehen, welcher einer Verstopfung oder einer Blasenbildung in einem durch die Pumpkammer und die Einlaß- und die Auslaßleitung festgelegten Flüssigkeitsvolumen entspricht.

2. Pumpvorrichtung nach Anspruch 1, bei welcher das Feststellungsmittel Verstopfungsfeststellungsmittel umfaßt, welche zum Vergleichen der erfaßten Amplitude der Auslenkung mit einem unteren Schwellenniveau betreibbar sind und das Vorliegen einer Verstopfung anzeigen, wenn die erfaßte Amplitude kleiner ist als das untere Schwellenniveau.

3. Pumpvorrichtung nach Anspruch 1 oder 2, bei welcher das Feststellungsmittel Blasenfeststellungsmittel umfaßt, welche zum Vergleichen der erfaßten Amplitude der Auslenkung mit einem oberen Schwellenniveau betreibbar sind und das Vorliegen einer Blase anzeigen, wenn die erfaßte Amplitude größer ist als das obere Schwellenniveau.

4. Pumpvorrichtung nach einem der Ansprüche 1 bis 3, umfassend mit dem elektronischen Schaltungsmittel verbundene Förderleistungserfassungsmittel und wobei das Schaltungsmittel das piezoelektrische Element mit einer Frequenz erregt, welche veränderbar ist, um die erfaßte Förderleistung auf einem vorbestimmten Niveau zu halten.

5. Pumpvorrichtung nach einem der vorhergehenden Ansprüche, bei welcher das elektrische Schaltungsmittel in einem von dem Pumpkörper abnehmbaren Gehäuse (38) angeordnet ist und das Gehäuse in Betrieb durch lösbare Verbindungsmittel (39) mit dem Pumpkörper verbunden ist.

6. Pumpvorrichtung nach einem der vorhergehenden Ansprüche, bei welcher das piezoelektrische Element in dem Gehäuse an einer Stelle angeordnet ist, welche in Betrieb, wenn das Gehäuse und der Pumpkörper betriebsmäßig verbunden sind, über der flexiblen Wandung des Pumpkörpers liegt, und bei welcher die Vorrichtung ein Vorspannmittel (103) umfaßt, welches die Wandung in Betriebseingriff mit dem Wandler vorspannt.

7. Pumpvorrichtung nach Anspruch 6, bei welcher das Vorspannmittel einen ringförmigen Membranabschnitt (103) der flexiblen Wandung umfaßt, welcher umfangsmäßig mit einem festen Abschnitt (105) der flexiblen Wandung verbunden ist und ein Formgedächtnis aufweist, so daß dann, wenn der Pumpkörper betriebsmäßig mit dem Gehäuse verbunden ist, der feste Abschnitt in Eingriff mit dem Wandler vorgespannt ist.

8. Pumpvorrichtung nach einem der Ansprüche 5 bis 7, bei welcher der Körper integral mit einem Speicher (129) geformt ist, welcher in Betrieb einen Fluidvorrat aufnimmt, wobei der Speicher mit der Einlaßleitung in Verbindung stehend angeschlossen ist.

9. Pumpvorrichtung nach Anspruch 8, bei welcher das Gehäuse ein Speicherfach (121) umfaßt, welches den Speicher aufnimmt, wenn das Gehäuse und der Körper betriebsmäßig verbunden sind, und Verschlußmittel (130) für das Speicherfach, wobei die Vorrichtung ferner Vorbereitungsmittel umfaßt, welche wahlweise betätigbar sind, um das erste und das zweite Ventil in den offenen Zustand vorzuspannen, um ein Vorbereiten der Kammer zu ermöglichen, wobei die Vorbereitungsmittel durch einen manuell betriebenen Griff (33) betätigt werden, welcher an dem Gehäuse angebracht ist, und bei welcher der Griff und die Verschlußmittel gegenseitig verriegeln, um einen Zugang zu dem Speicherfach zu verhindern, wenn sich der Griff in einer Stellung befindet, welche einer Nicht-Betätigung der Vorbereitungsmittel zum Vorsehen des Normalbetriebs der Ventile entspricht.

## Revendications

1. Appareil formant pompe (1, 100, 120) destinée à être utilisée pour le pompage d'un liquide en vue de son infusion dans le corps d'un être humain ou d'un animal, l'appareil comportant un corps de pompe (2) définissant une chambre de pompe (6), un conduit d'entrée (15) communiquant avec la chambre de pompe en vue de son raccordement à une source (129) d'un liquide d'infusion à pomper, de sorte que, en service, le liquide remplit la chambre de pompe, un conduit de sortie (20) communiquant avec la chambre en vue du refoulement du liquide lorsque celui-ci est pompé, des moyens d'actionnement (10, 50) aptes à agir pour faire varier le volume de la chambre de pompe par le déplacement d'un paroi flexible (9) de la chambre de pompe pour pomper le liquide à partir de cette dernière, des première et seconde soupapes (13, 18) situées dans les conduits d'entrée et de sortie, respectivement, les moyens d'actionnement comportant un élément piézoélectrique (10, 50) ayant au moins une électrode de détection (63, 64) et des moyens formant circuit électronique (40) aptes à agir pour exciter l'élément piézoélectrique par impulsions, caractérisé en ce que chaque soupape étant constituée par une soupape de retenue apte à agir en réponse à une pression ou à une aspiration engendrée dans la chambre de pompe et l'électrode de détection au nombre d'au moins une (63, 64) détectant l'amplitude de la déformation de la paroi flexible (9), les moyens électroniques formant circuit comprenant des moyens de détection comparant l'amplitude de déformation détectée à au moins un niveau de seuil pour fournir une indication d'une anomalie de fonctionnement de l'appareil formant pompe susceptible de traduire une occlusion ou la formation de bulles dans un volume du liquide défini par la chambre de pompe et les conduits d'entrée et de sortie.

2. Appareil formant pompe selon la revendication 1, dans lequel les moyens de détection comprennent des moyens de détection d'occlusion aptes à agir pour comparer l'amplitude de déformation détectée à un niveau de seuil inférieur et à signaler la présence d'une occlusion lorsque l'amplitude détectée est inférieure au niveau de seuil inférieur.

3. Appareil formant pompe selon la revendication 2 ou la revendication 2, dans lequel les moyens de détection comprennent des moyens de détection de bulles aptes à agir pour comparer l'amplitude détectée de la déformation à un niveau de seuil supérieur et signalant la présence d'une bulle lorsque l'amplitude détectée est supérieure au niveau de seuil supérieur.

4. Appareil formant pompe selon l'une quelconque des revendications 1 à 3, comprenant des moyens de détection de débit reliés au moyens formant circuit électronique et dans lequel les moyens formant circuit excitent l'élément piézoélectrique suivant une fréquence qui est variable pour maintenir le débit détecté à un niveau prédéterminé.

5. Appareil formant pompe selon l'une quelconque des revendications précédentes, dans lequel les moyens formant circuit électronique sont situés dans un boîtier (38) qui est apte à être séparé du corps de pompe et le boîtier est raccordé, en service, au corps de pompe à l'aide de moyens de raccordement libérables (39).

6. Appareil formant pompe selon l'une quelconque des revendications précédentes, dans lequel l'élément piézoélectrique se trouve dans le boîtier en un emplacement qui recouvre la paroi flexible du corps de pompe, en service, lorsque le boîtier et le corps de pompe sont reliés en relation opérationnelle, et dans lequel l'appareil comprend des moyens de sollicitation (103) sollicitant la paroi pour la mettre en contact de travail avec le transducteur.

7. Appareil formant pompe selon la revendication 6, dans lequel les moyens de polarisation comprennent une partie formant membrane annulaire (103) de la paroi flexible raccordée par la périphérie à une partie rigide (105) de la paroi flexible et ayant une mémoire de forme telle que, lorsque le corps de pompe est raccordé opérationnellement au boîtier, la partie rigide soit sollicitée de manière à porter contre le transducteur.

8. Appareil formant pompe selon l'une quelconque des revendications 5 à 7, dans lequel le corps est formé de manière à faire partie intégrante d'un réservoir (129) recevant, en service, une alimentation en fluide, le réservoir étant mis en communication avec le conduit d'entrée.

9. Appareil formant pompe selon la revendication 8, dans lequel le boîtier comprend un compartiment à réservoir (121) recevant le réservoir lorsque le boîtier et le corps sont raccordés opérationnellement et des moyens de fermeture (130) pour le compartiment à réservoir, l'appareil comprenant en outre des moyens d'amorçage aptes à agir sélectivement pour solliciter les première et seconde soupapes vers une condition ouverte pour permettre l'amorçage de la chambre, les moyens d'amorçage étant actionnés par une poignée à commande manuelle (33) montée sur le boîtier, et la poignée et les moyens de fermeture se verrouillant ensemble pour interdire l'accès au compartiment à réservoir lorsque la poignée se trouve dans une position correspondant à la désactivation des moyens d'amorçage pour permettre le fonctionnement normal des soupapes.
